(19) Europäisches Patentamt
European Patent Office
Office européen des brevets

(11) EP 2 250 267 B1

(12) # FASCICULE DE BREVET EUROPEEN

(45) Date de publication et mention de la délivrance du brevet:
**14.09.2016 Bulletin 2016/37**

(21) Numéro de dépôt: **09707000.7**

(22) Date de dépôt: **30.01.2009**

(51) Int Cl.:
***C12N 15/11*** (2006.01) ***C07H 21/02*** (2006.01)
***A61K 31/7088*** (2006.01) ***A61K 48/00*** (2006.01)

(86) Numéro de dépôt international:
**PCT/IB2009/050379**

(87) Numéro de publication internationale:
**WO 2009/095887 (06.08.2009 Gazette 2009/32)**

(54) **siRNA CATIONIQUES, SYNTHÈSE ET UTILISATION POUR L'ARN INTERFÉRENCE**

KATIONISCHE siRNA, SYNTHESE UND VERWENDUNG FÜR INTERFERIERENDE RNA

CATIONIC SIRNAS, SYNTHESIS AND USE FOR INTERFERING RNA

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **30.01.2008 FR 0800492**

(43) Date de publication de la demande:
**17.11.2010 Bulletin 2010/46**

(73) Titulaires:
- **Centre National de la Recherche Scientifique 75794 Paris Cedex 16 (FR)**
- **Université de Strasbourg 67081 Strasbourg Cedex (FR)**

(72) Inventeurs:
- **BEHR, Jean-Paul 66660 PORT-VENDRES (FR)**
- **KOTERA, Mitsuharu 67000 Strasbourg (FR)**
- **PFENDER, Nadège 67640 Lispheim (FR)**
- **REMY, Jean-Serge F-67370 Schnersheim (FR)**

(74) Mandataire: **Paris, Fabienne**
**Ernest Gutmann - Yves Plasseraud S.A.S.**
**3, rue Auber**
**75009 Paris (FR)**

(56) Documents cités:
**WO-A-2006/052854 WO-A-2007/069092**

- **MOSCHOS STERGHIOS ATHANASIOS ET AL: "Lung delivery studies using siRNA conjugated to TAT(48-60) and penetratin reveal peptide induced reduction in gene expression and induction of innate immunity." BIOCONJUGATE CHEMISTRY 2007 SEP-OCT, vol. 18, no. 5, septembre 2007 (2007-09), pages 1450-1459, XP002492909 ISSN: 1043-1802 cité dans la demande**
- **MURATOVSKA ALEKSANDRA ET AL: "Conjugate for efficient delivery of short interfering RNA (siRNA) into mammalian cells." FEBS LETTERS 30 JAN 2004, vol. 558, no. 1-3, 30 janvier 2004 (2004-01-30), pages 63-68, XP002492910 ISSN: 0014-5793**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 250 267 B1

## Description

La demande et l'invention se rapportent à des siRNA (pour small interfering ribonucleic acid) cationiques, à leur synthèse automatisée ainsi qu'à leurs applications biologiques basées sur leurs propriétés de pénétration intracellulaire et mettant en jeu le mécanisme d'ARN interférence.

L'ARN interférence est un mécanisme intracellulaire permettant le contrôle séquence-spécifique de l'expression d'un gène endogène ou exogène. Les médiateurs de ce mécanisme sont de petites hélices d'acide ribonucléique bicaténaire d'environ 2 tours, les siRNA, de séquence identique à un fragment du gène-cible. Bien que l'ARN interférence puisse aboutir à la modification permanente du gène-cible dans la chromatine d'une cellule, l'effet le plus immédiat de l'introduction d'un siRNA dans une cellule est l'inactivation de l'ARN messager-cible. Les siRNA sont généralement constitués de deux oligoribonucléotides 19-mères de séquences complémentaires comportant des extensions 3'-dTdT, mais certains appariements imparfaits ou des séquences jusqu'à 27-mères peuvent également être efficaces.

La disparition d'une protéine ou la destruction sélective d'un ARN viral peuvent avoir des applications thérapeutiques d'intérêt majeur dans des domaines aussi variés que la cancérologie, l'allergie ou les maladies infectieuses. Malheureusement, les cellules animales intactes sont imperméables aux acides nucléiques, comme le montrent les tentatives infructueuses d'introduction de siRNA tels quels dans des cellules en culture ou in vivo. L'expérimentation animale montre néanmoins que le foie, un organe capable de régénérer rapidement ses cellules, ou bien les cellules musculaires, qui sont particulièrement grandes et robustes, peuvent capter des siRNA et survivre à la rupture de leur membrane cellulaire induite par le processus d'injection. Mais l'injection intraveineuse rapide d'un grand volume de liquide ou le caractère très localisé de l'injection intramusculaire ne sont pas compatibles avec l'utilisation en thérapie humaine.

La transfection de siRNA dans le cytoplasme de cellules -sans rupture de la membrane cellulaire- peut être obtenue après mélange avec un excès de transporteur cationique. Des macromolécules polycationiques comme la polyéthylènimine ou des agrégats de lipides cationiques sont capables de jouer ce rôle de vecteur (1). Les molécules de siRNA polyanioniques s'aggrègent en effet avec un excès de transporteur en formant un fin précipité cationique. Ces particules permettent la transfection efficace de siRNA dans un grand nombre de cellules en culture par l'intermédiaire d'héparanesulfates anioniques présents à la surface des cellules (2).

Dans un animal, il n'en va plus de même. Injectées dans le sang, les particules sont captées et digérées par les cellules qui phagocytent les particules microbiennes. Injectées dans un tissu ou une tumeur, leur taille (100-1000nm) empêche leur diffusion vers les cellules situées au-delà du site d'injection. Des travaux récents de (3) et (4) montrent néanmoins qu'il est possible d'éviter les cellules phagocytaires du sang sans inhiber totalement la fixation cellulaire, en recouvrant les particules par des résidus de polyéthylèneglycol (particules furtives).

Ces considérations ont conduit les inventeurs à envisager la synthèse de siRNA intrinsèquement cationiques, c'est-à-dire de molécules de siRNA modifiées où les charges cationiques compenseraient les charges anioniques portées par les phosphates de l'acide nucléique. Ces composés pourraient éventuellement se passer d'un vecteur pour pénétrer dans la cellule et par conséquent le médicament aurait l'avantage d'être moléculaire au lieu d'être particulaire.

Afin de ne perturber ni la fixation du siRNA au complexe protéique qui détruit l'ARN messager, ni la reconnaissance de ce dernier par hybridation, les modifications chimiques ne sont possibles qu'aux extrémités de l'acide nucléique.

De très nombreux motifs chimiques ont été greffés par le passé aux extrémités d'oligonucléotides, dans le but d'augmenter leur pénétration cellulaire à des fins de thérapie par les approches antisens. Quelques articles publiés très récemment font état de tentatives similaires dans le domaine des siRNA.

Un premier article paru en 2006 fait état de la conjugaison de polyéthylèneglycol à un siRNA à travers un pont disulfure, mais l'internalisation cellulaire nécessite tout de même l'association non-covalente par mélange avec un polymère cationique (5).
En 2007, 2 articles décrivent l'utilisation de siRNA (40 charges anioniques) conjugués à divers peptides cationiques comportant 7 ou 8 charges cationiques. Ces siRNA restent très anioniques et de l'aveu des auteurs eux-mêmes, la pénétration intracellulaire et l'efficacité ne sont pas augmentées (6) et (7).
Par ailleurs, WO 2006/052854 décrit la conjugaison d'une spermine à des siRNAs.

Le greffage chimique direct d'un polycation préformé à un oligonucléotide comme décrit dans les articles précédents est possible, mais cause des problèmes de précipitation et peut rendre la caractérisation imprécise à cause de la polydispersité du polymère cationique dès que l'on manipule des polycations comportant un nombre de charges comparable à celui de l'oligonucléotide. Les inventeurs ont surmonté les problèmes ci-dessus en s'appuyant sur une technique de synthèse séquentielle de l'oligocation par une chimie de conjugaison identique à celle utilisée pour la synthèse de la séquence oligoribonucléotidique elle-même. La synthèse automatique de conjugués oligodeoxyribonucléotides-oligospermines a fait l'objet d'une précédente demande internationale PCT (8).

Des modifications importantes ont été apportées à la synthèse/purification/caractérisation/conservation des composés décrits dans la précédente demande PCT, la présence du groupement 2'-OH rendant la synthèse plus difficile et surtout rendant les composés plus instables : sa déprotection implique une étape supplémentaire - des traces de

RNases dégradent l'ARN - les fonctions ammonium/amine des spermines sont des catalyseurs acidobasiques provoquant l'hydrolyse des conjugués oligoribonucléotide-oligospermine.

La demande et l'invention visent donc à fournir des siRNA intrinsèquement cationiques.

Elles visent également une méthode de synthèse de ces siRNA cationiques qui puisse être étendue à l'échelle industrielle en vue de la production GMP.

Selon un autre aspect, la demande et l'invention visent aussi les applications des nouveaux siRNA dans les domaines pharmaceutiques et biotechnologiques.

Les siRNA cationiques de l'invention sont caractérisés en ce qu'il s'agit de fragments d'ARN double brin, aux extrémités desquels sont greffés des oligocations, le nombre de charges cationiques greffées étant supérieur à celui des charges anioniques des phosphates internucléosidiques des brins d'ARN, et caractérisés en ce que lesdits oligocations sont des oligospermines greffées en 3' et/ou 5' du brin sens et/ou en 3' du brin antisens du siRNA conformément à la formule

$$(N)_i\text{-}[\overset{-}{PO_3}\text{-}(CH_2)_4\text{-}\overset{+}{NH_2}\text{-}(CH_2)_3\text{-}\overset{+}{NH_2}\text{-}(CH_2)_4\text{-}\overset{+}{NH_2}\text{-}(CH_2)_3\text{-}\overset{+}{NH_2}\text{-}(CH_2)_4\text{-}O]_jH$$

dans laquelle $(N)_i$ représente un oligoribonucléotide i-mère, avec i= 15 à 30 ainsi que toutes modifications chimiques ou substitutions conservant la forme A de la double hélice du siRNA et dans laquelle j = 1 à 50.

Les siRNA cationiques de la demande ou de l'invention peuvent être caractérisés en ce qu'il s'agit de fragments d'ARN double brin, aux extrémités desquels sont greffés des oligocations, le nombre total de charges cationiques greffées étant comparable ou supérieur à celui des charges anioniques portées par les phosphates internucléosidiques des brins d'ARN.

De manière inattendue, et comme démontré par les résultats donnés dans les exemples, de tels siRNA cationiques, sont capables d'induire un phénomène d'ARN interférence en l'absence de tout agent de transfection. La démonstration est réalisée dans un environnement contrôlé, sur des cellules en culture, où des conjugués siRNA-oligopeptides cationiques restant globalement anioniques n'ont pas montré d'effet (vide supra).

Par « ARN double brin », on entend une double hélice d'acide nucléique de la famille A induite par la présence d'un atome autre que H en position 2' du ribose (par opposition à l'ADN qui est une hélice B).

La charge globale Σ du siRNA cationique, c'est-à-dire la somme totale des charges cationiques greffées chimiquement diminuée de la somme totale des charges anioniques des groupements phosphates internucléosidiques, est de -30 à +50. En effet, des siRNA (charge -40) greffés à un peptide TAT de séquence SEQ ID N°1 GRKKRRQRRRPPQC (charge +9 ; Σ = -31) ou à un peptide pénétratine de séquence SEQ ID N°2 RQIKIWFQNRRMKWKKC (charge +8 ; Σ = -32) ne sont pas capables d'induire le phénomène d'ARN interférence sans vecteur (9).

La complémentarité des bases de la double hélice d'ARN peut être parfaite ou partielle.

Dans une variante de réalisation, chaque oligoribonucléotide présente une extension simple brin en 3' de la double hélice.

Dans une autre variante de la demande, les oligocations sont greffés en 3' ou/et 5' du brin sens ou/et en 3' du brin antisens, le brin antisens étant celui qui guide le complexe protéique responsable de la dégradation de l'ARN messager. Le greffage en position 5' du brin antisens abolit l'effet d'ARN interférence.

De manière préférée, les siRNA cationiques objet de la demande ou l'invention sont constitués de fragments d'ARN double brin longs de 15 à 30 ribonucléotides, en particulier 19 à 30 ribonucléotides, aux extrémités desquels sont greffés, par liaison covalente, 1 à 3 oligocations dont le nombre de charges cationiques cumulées est comparable ou supérieur au nombre de charges anioniques portées par l'ARN.

La demande et l'invention visent plus particulièrement des siRNA cationiques dans lesquels un brin au moins répond à la formule (I)

Ni-Aj ou Aj-Ni (I)

dans laquelle

- Ni représente un oligoribonucléotide i-mère, avec i= 15 à 30, en particulier 19 à 30, ainsi que toutes modifications chimiques ou substitutions conservant la forme A de la double hélice du siRNA,
- Aj représente un oligocation j-mère, avec j = 1-50, A répondant

- à la formule (II)

$$-\ HPO_3R^1\text{-}(X\text{-}R^2)_{n1}\text{-}X\text{-}R^3\text{-}O\text{-} \qquad (II),$$

avec $R^1$, $R^2$ et $R^3$, identiques ou différents, représentant un radical alkylène inférieur, X étant NH ou $NC(NH_2)_2$, n1 = 2 à 20, ou

- à la formule (III)

$$-HPO_3\text{-}R^4\text{-}CH(R^5X^1)\text{-}R^6\text{-}O\text{-}, \qquad \text{(III)}$$

avec $R^4$, $R^5$ et $R^6$, identiques ou différents, représentant un radical alkylène inférieur et $X^1$ étant choisi parmi la putrescine, la spermidine ou la spermine, ou

- à la formule (IV)

$$-HPO_3\text{-}R^7\text{-}(aa)_{n2}\text{-}R^8\text{-}O\text{-}, \qquad \text{(IV)}$$

avec $R^7$ et $R^8$ identiques ou différents, représentant un radical alkylène inférieur, $(aa)_{n2}$ étant un peptide contenant des acides aminés naturels avec des chaînes latérales cationiques comme l'arginine, la lysine, l'ornithine, l'histidine, l'acide diaminopropionique et n2= 2 à 20.

Par « radical alkylène inférieur », on entend, dans la description et les revendications, un radical alkylène en C1-C5, linéaire, branché ou substitué.

Dans un mode préféré de réalisation de la demande ou l'invention, les oligocations sont des oligoamines. Ces oligoamines sont avantageusement choisies dans le groupe comprenant la spermine, la spermidine ou la putrescine.

Des siRNA cationiques correspondants sont avantageusement des oligonucléotides-oligospermines de structure (V)

$$(N)_i\text{-}[\overset{-}{PO_3}\text{-}(CH_2)_4\text{-}\overset{+}{NH_2}\text{-}(CH_2)_3\text{-}\overset{+}{NH_2}\text{-}(CH_2)_4\text{-}\overset{+}{NH_2}\text{-}(CH_2)_3\text{-}\overset{+}{NH_2}\text{-}(CH_2)_4\text{-}O]_jH \quad \text{(V)}$$

dans laquelle N, i et j sont tels que définis ci-dessus.

Les domaines d'application de l'ARN interférence sont grandissants. Les siRNA sont utilisés par l'industrie pharmaceutique et biotechnologique pour établir un lien entre gènes, maladies et molécules potentiellement actives contre ces maladies; ce sont essentiellement des techniques de criblages haut débit sur des cellules animales. Les siRNA cationiques de la demande ou l'invention permettent de simplifier le criblage, et surtout d'étendre la technique à des animaux où physiologie, biodistribution, excrétion, seraient prises en compte dans la validation d'un composé en phase préclinique, augmentant ainsi les chances de succès et réduisant les coûteux échecs en phase clinique.

Le plus grand potentiel des siRNA se trouve néanmoins dans leur utilisation directe en tant que médicaments pour inhiber la biosynthèse d'un ARN ou d'une protéine dans les cellules d'un patient. Des pathologies très diverses comme le cancer, les infections virales, l'asthme ou encore les maladies auto-immunes deviennent alors curables.

La demande et l'invention couvrent par conséquent l'utilisation de siRNA cationiques pour ces applications.

La demande et l'invention visent ainsi les siRNA cationiques définis ci-dessus pour une utilisation comme médicaments.

Plus particulièrement, la demande et l'invention visent des compositions pharmaceutiques caractérisées en ce qu'elles renferment une quantité efficace d'au moins un siRNA de la demande ou l'invention, respectivement, tel que défini ci-dessus, en association avec un véhicule pharmaceutiquement inerte.

Elles visent également l'application desdits siRNA cationiques en biologie moléculaire, génomique fonctionnelle ainsi que dans les techniques de criblage.

La demande et l'invention visent également un procédé de synthèse automatisée d'un brin de siRNA cationique, caractérisé en ce qu'il comprend : le couplage séquentiel 3' vers 5' de ribonucléotides sur support solide, précédé et/ou suivi du couplage séquentiel de synthons cationiques, le brin répondant à la formule (VI)

$$dT\text{-}dT\text{-}(N)_iA_j \text{ ou } A_j\text{-}dT\text{-}dT\text{-}(N)_i \qquad \text{(VI)}$$

avec N, i, A, et j tels que définis ci-dessus

La demande et l'invention visent plus particulièrement un procédé de synthèse de siRNA cationiques dans lesquels les synthons cationiques sont des oligoamines phosphoramidites et répondent

- à la formule (VII)

$$P(OR^9)(N(R^{10})_2)\text{-}O\text{-}R^1\text{-}(X\text{-}R^2)_{n1}\text{-}X\text{-}R^3\text{-}O\text{-}Prot, \qquad (VII)$$

dans laquelle $R^1$, $R^2$, $R^3$, et ni sont tels que définis ci-dessus, X est un groupe NH ou $NC(NH_2)_2$ protégé, $R^9$ est $-CH_2CH_2CN$, ou un radical alkylène inférieur, $R^{10}$ est un radical alkylène inférieur, ou $-N(R^{10})_2$ est un radical pyrrolidino, pipéridino ou morpholino et Prot est un groupe protecteur utilisé en synthèse oligonucléotique, tel que DMT, MMT; ou

- à la formule (VIII)

$$P(OR^9)(N(R^{10})_2)\text{-}O\text{-}R^4\text{-}CH(R^5X^1)\text{-}R^6\text{-}O\text{-}Prot, \qquad (VIII)$$

dans laquelle $R^4$, $R^5$, $R^6$ identiques ou différents, représentent radical alkylène inférieur, $X^1$ est une putrescine, spermidine ou spermine avec des groupements protecteurs appropriés, $R^9$ et $R^{10}$ sont tels que définis ci-dessus; ou à la formule (IX)

$$P(OR^9)(N(R^{10})_2)\text{-}O\text{-}R^7\text{-}(aa)_{n2}\text{-}R^8\text{-}O\text{-}Prot, \qquad (IX)$$

dans laquelle $R^7$, $R^8$, $R^9$, $R^{10}$, n2, et Prot sont comme défini ci-dessus, $(aa)_{n2}$ est un peptide contenant des acides aminés naturels avec des chaînes latérales cationiques protégés, comme l'arginine, la lysine, l'ornithine, l'histidine, l'acide diaminopropionique et n2= 2 to 20.

Dans un mode préféré de la demande ou l'invention l'oligoamine est une oligospermine

D'autres caractéristiques et avantages de la demande ou l'invention sont donnés dans les exemples qui suivent.

Les caractéristiques de synthèse et de purification de conjugués oligoribonucléotide-oligospermine ainsi que les propriétés d'ARN interférence des siRNA correspondants sont détaillées dans ces exemples, sans limiter l'invention à ces seuls composés. En particulier, la synthèse d'un 21 -mère ciblant la luciférase et comportant jusqu'à 30 motifs spermine est décrite. Lorsque le nombre de charges cationiques avoisine le nombre de phosphates portés par le siRNA correspondant, on observe une extinction particulièrement importante et sélective du gène de la luciférase dans une lignée cellulaire qui exprime constitutivement ce gène.

Dans les exemples, il sera fait référence aux Figures 1 à 6, qui représentent respectivement :

Figure 1 : Analyse HPLC montrant la dégradation de l'oligonucléotide <GL3ss>$S_5$ dans l'eau.

Figure 2: Profil HPLC des oligonucléotides <GL3ss>$S_n$, avec n = 0, 1, 3, 5, et <GL3as>$S_n$, avec n = 0, 1.

Figure 3 : Spectres de masses MALDI-TOF.

Figure 4 : Extinction du gène de la luciférase en fonction du nombre de spermines.

Figure 5 : Extinction du gène de la luciférase en fonction de la concentration du siRNA.

Figure 6 : Contrôles de spécificité.

<u>Exemple 1 : Synthèse, purification et caractérisation d'oligonucléotides 21-mère comportant **n** spermines, de formule :</u>

$^{3'}$dTdT - AGCUUCAUGAGUCGCAUUC$^{5'}$ - O 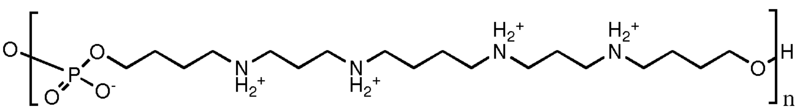

<GL3ss>$S_n$, où <GL3ss> est un oligonucléotide 21-mère, S est un résidu phosphospermine, n = 0, 1, 3, 5, 20, 30

*Synthèse automatisée* : une série d'oligonucléotides 21 mères <GL3ss> protégés, de séquence SEQ ID N°3 $^{3'}$dTdT- A G C U U C A U G A G U C G C A U U C$^{5'}$ dont la partie ribonucléotidique, correspondant aux nucléotides 153 à 171 du gène de la luciférase *Photinus pyralis* présent dans le plasmide GL3 (Proméga), a été synthétisée en phase

solide en utilisant la chimie des phosphoramidites β-cyanoéthyles (CE), sur un synthétiseur Expedite. La résine comportait déjà 2 deoxythymidines préalablement greffées. La synthèse a été poursuivie avec n cycles utilisant le synthon spermine phosphoramidite. Les composés de structure ci-dessous (n= 0, 1, 3, 5, 20, 30), fixés sur la résine, ont été obtenus.

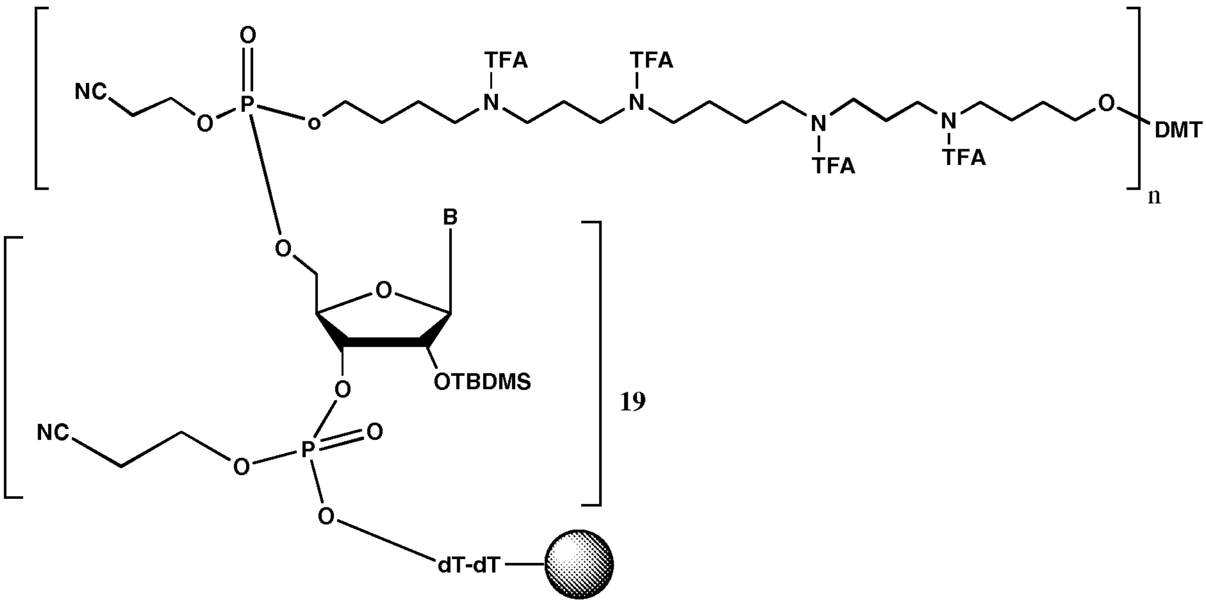

19

Les phosphoramidites CE, les supports Ultramild et les réactifs généraux utilisés pour la synthèse automatisée proviennent de Glen Research (Eurogentec). La phosphoramidite spermine provient de Novalyst. Les réactifs Ultramild permettent la déprotection des groupements protecteurs des bases nucléiques et le clivage simultané de l'oligo du support solide dans des conditions alcalines plus douces. Les monomères utilisés, à savoir A protégé par le groupement phénoxyacétyle (Pac-ACE), G protégé par le groupement 4-isopropyl-phénoxyacétyle ((iPr-Pac)-G-CE) et C protégé par un groupement acétyle (Ac-C-CE) ont permis d'éviter la dégradation des composés au cours du work-up. La position 2'-OH des nucléotides a été protégée par le groupement protecteur tertiobutyldiméthylsilyle TBDMS.

La synthèse automatisée a été réalisée dans les conditions de couplage standard, excepté dans le cas du couplage de la phosphoramidite spermine qui a nécessité un temps de réaction plus long (20 minutes) et une solution en phosphoramidite plus concentrée (0,1 M soit 120 mg par ml d'acétonitrile anhydre). Dans le cas du couplage de 20 et 30 spermines, une double réaction de couplage a été nécessaire dans le cas des 4 premières spermines.

Les fractions contenant le cation coloré diméthoxytrityle libéré à chaque étape de la synthèse ont été collectées, diluées et analysées par spectrophotométrie afin de déterminer les rendements des étapes de couplage. Pour les couplages des quatre nucléotides naturels (A, U, C et G), les rendements excédaient 97%, tandis que les rendements de couplage de la phosphoramidite spermine se trouvaient entre 90 et 98%.

De manière analogue, ont été synthétisées

- une séquence-contrôle négatif comportant 20 spermines SEQ ID N°4, dont la séquence est issue de la région correspondante de la luciférase du plasmide GL2 (Proméga) $^{3'}$dTdT- A G C U U C A U A A G G C G C A U G C$^{5'}$. L'oligonucléotide <GL2ss>$S_{20}$ comporte 3 mismatchs par rapport à la séquence homogogue de GL3 et de ce fait le siRNA correspondant n'a pas la capacité d'interférer avec l'ARN messager de la luciférase GL3 produit par les cellules. - une séquence antisens SEQ ID N°5 $^{3'}$dTdT-G A A U G C G A C U C A U G A A G C U$^{5'}$ comportant une spermine en 5'. Ici, c'est l'autre brin <GL3as>$S_1$ de la double hélice de siRNA qui porte la partie cationique.

| | Rendement de couplage moyen des spermines |
|---|---|
| <GL3ss>$S_1$ | 98% |
| <GL3ss>$S_3$ | 97% |
| <GL3ss>$S_5$ | 95% |
| <GL3ss>$S_{20}$ | 92% |

(suite)

| | Rendement de couplage moyen des spermines |
|---|---|
| <GL3ss>$S_{30}$ | 90% |
| <GL3as> $S_1$ | 98% |
| <GL2ss>$S_{20}$ | 90% |

Dans tous les cas, la stratégie DMT-ON a été utilisée, conservant le groupement DMT en position 5' finale des oligomères afin de faciliter la purification.

*Traitement post-synthétique :* Après la synthèse automatisée, les oligomères ont été libérés du support solide et déprotégés simultanément dans les conditions Ultramild standard, par traitement à l'ammoniaque 28% à température ambiante, pendant une nuit.

Après lyophilisation, la seconde étape a consisté en la déprotection du groupement TBDMS en position 2'-OH, à l'aide d'une solution 1.0 M de fluorure de tetrabutylammonium TBAF dans le THF (Aldrich). Les oligonucléotides ont été traités par 50 équivalents de TBAF par groupement TBDMS pendant 15h, à température ambiante, dans l'obscurité.

*Purification :* Les oligonucléotides ont été purifiés en utilisant des colonnes Poly-Pak II™ (Glen Research/Eurogentec) d'après les instructions données par le fournisseur. L'élution finale était réalisée à l'aide d'un mélange acétonitrile/eau (50/50) dans tous les cas, excepté pour les oligonucléotides comportant 20 et 30 spermines qui ont nécessité l'utilisation d'acétonitrile/ammoniaque aqueux 28% dilué au 1/20e (20/80). Les fractions contenant l'oligonucléotide ont été révélées en en posant une goutte sur une plaque de silice couche mince fluorescente et ont été immédiatement réunies et lyophilisées afin d'éliminer les solvants et éviter la dégradation. Une poudre blanche a ainsi été obtenue.

La manipulation d'oligoribonucléotides a de plus nécessité l'exclusion minutieuse des sources de ribonucléases. Il a donc été essentiel de porter des gants afin d'éviter la contamination par les RNAses présentes sur les mains. Du matériel stérile (pipettes, tubes Eppendorf) a été utilisé, le tout sous une hotte à flux laminaire.
Au départ, les échantillons d'ARN ont été solubilisés dans l'eau et conservés au congélateur. Si les brins non modifiés sont restés stables, et ce même après plusieurs mois dans ces conditions, l'analyse HPLC a montré que les oligoribonucléotides-oligospermines se sont dégradés très rapidement (Figure 1, c.f. conditions HPLC ci-dessous).

Les oligonucléotides ont donc été solubilisés dans un tampon phosphate pH 5,6 (50mM) et conservés à -20°C ce qui a permis d'éviter la dégradation des composés par hydrolyse catalysée par les spermines, et ce même après 3 mois (suivi HPLC).

La concentration des oligonucléotides a été déterminée en utilisant le coefficient molaire d'extinction suivant (260nm, $mol^{-1}\ dm^3\ cm^{-1}$):

$$\varepsilon = (15{,}4\ N_A + 11{,}7\ N_G + 7{,}4\ N_C + 8{,}8\ N_U) \times 0{,}9 \times 10^3$$

Les oligonucléotides de charge globalement négative comportant respectivement 0, 1, 3, et 5 spermines ont été analysés par HPLC échangeuse d'anions (Macherey Nagel SAX 1000-8) avec un gradient linéaire de B allant de 0 à 100% en 15 minutes (A : $KH_2PO_4$ 20mM, AcCN 20% ; B : A, NaCl 1M). Les profils HPLC sont montrés en Figure 2.

Les oligonucléotides ont été solubilisés dans 500µl d'eau distillée. L'échantillon et la matrice d'acide betahydroxypyruvique ont été mélangés ensemble sur la plaque. Une fois cristallisé, l'échantillon a été analysé sur un appareil BRUKER Ultrafex. Les spectres de masses sont donnés en Figure 3.

| | Masse attendue (g/mole) | Masse observée |
|---|---|---|
| <GL3ss> | 6606,9 | 6612,2 |
| <GL3ss>$S_1$ | 7015,5 | 7031,2 |
| <GL3ss>$S_3$ | 7832,5 | 7857,9 |
| <GL3 as> | 6693,9 | 6740,2 |
| <GL3 as> $S_1$ | 7101,6 | 7119,5 |
| <GL2 ss> | 6669,0 | 6678,2 |

Exemple 2 : ARN interférence ; extinction du gène de la luciférase par les siRNA cationiques. Effet du nombre de charges cationiques.

*Culture cellulaire :* La lignée cellulaire A549Luc provient de cellules épithéliales bronchiques humaines (CCL-185, ATCC) par transfection stable du plasmide pGL3 (Proméga).

Les cellules A549Luc ont été mises en culture dans du milieu RPMI 1640 complet (Eurobio) additionné de 10% de sérum de veau foetal (FCS, Perbio), 2 mM de glutamax (Eurobio), 100 U/ml de pénicilline (Eurobio) et de 100µg/ml de streptomycine (Eurobio) et incubées à 37°C, 5% de $CO_2$ dans une atmosphère saturée en humidité.

Un jour avant la transfection des siRNA, les cellules ont été décollées par de la trypsine (Eurobio) et resuspendues dans du milieu RPMI/FCS auquel 16µl/ml d'antibiotique G-418 (Proméga) ont été additionnés. Des plaques de 24 puits ont été préparées avec 25000 cellules par puits dans 1ml de milieu complet.

*Formation des duplex siRNA et incubation avec les cellules* : Dans un premier temps les duplex siRNA ont été préparés par mélange équimolaire de leur deux brins complémentaires, hybridation par chauffage à 70°C et refroidissement. Les concentrations étaient ajustées à des concentrations finales sur les cellules de 1, 10, ou 100 nM. Les duplex formés ont ensuite été dilués dans 100 µl du milieu RPMI.

Le contrôle positif de pénétration des siRNA dans les cellules a été effectué à l'aide de l'agent de transfection lipidique INTERFERin (Polyplus-transfection) utilisé selon les recommandations du fabricant. INTERFERin (2 µl) a été additionné au siRNA. Le mélange a été immédiatement homogénéisé et laissé au repos 10 minutes à température ambiante afin de favoriser la formation des complexes.

Pendant ce temps, le milieu de culture des cellules a été aspiré et les cellules adhérentes ont été lavées au tampon phosphate (PBS). Puis 500µl de RPMI ont été additionnés à chaque puits.

Les duplex siRNA (ou siRNA/INTERFERin) ont été ajoutés aux cellules (100µl/puits). La plaque a été homogénéisée par rotation manuelle. Chaque condition a été réalisée en triplica.

La plaque a été incubée à 37°C, 5% $CO_2$ durant 4h. Au bout de ce laps de temps, les cellules ont été observées au microscope pour vérifier l'absence de toxicité et 500µl de milieu RMPI additionné de 20% de FCS ont été ajoutés dans chaque puits.

La plaque a été incubée à l'étuve, à 37°C et 5%$CO_2$ durant 48h.

*Mesure de l'expression du gène de la luciférase :* Après 48h, les cellules ont été lavées au PBS puis lysées à l'aide d'une solution commerciale stock diluée 5x (Proméga). La plaque a ensuite été incubée 30 minutes à température ambiante. Les solutions ont été récupérées dans des microtubes et centrifugées 5 min à 1400 rpm à 4°C.

10µl de chaque échantillon ont été déposés sur une plaque de 96 puits. La plaque a été analysée à l'aide d'un luminomètre (Mediators PhL ou Berthold Centro LB690). Pour chaque échantillon, la lumière émise par la réaction catalysée par la protéine luciférase a été mesurée et normalisée sur une durée de 10 s.

*Dosage des protéines cellulaires totales :* Une gamme BSA (sérum albumine bovine) a préalablement été préparée afin de servir d'étalonnage. 15 µl de tampon de lyse ont été ajoutés par tube. 15µl de chaque échantillon à analyser ont été introduits dans des tubes. 3 blancs constitués de 15µl de tampon de lyse chacun ont été réalisés. 1ml de solution BCA (bicinchoninic acid) mix a été additionné dans chaque tube. Les mélanges obtenus ont ensuite été placés au bain marie à 60°C durant 30 minutes. L'absorbance a été lue à 562 nm.

*Evaluation de l'extinction du gène de la luciférase :* Les résultats ont d'abord été exprimés en RLU (Relative Light Unit) intégrés sur 10 secondes, normalisés par milligramme de protéines du lysat cellulaire. L'efficacité d'extinction de gène pour chaque condition a ensuite été exprimée en pourcentage de l'expression de la luciférase dans des cellules non incubées en présence de siRNA. Chaque barre représente la valeur moyenne du pourcentage d'expression du gène luciférase ainsi que sa déviation standard.

De haut en bas, la Figure 4 montre successivement que

- le siRNA <GL3ss><GL3as> formé des brins sens (ss) et antisens (as) de la séquence de la luciférase GL3 n'est pas actif ;
- ce même siRNA éteint l'expression de la luciférase lorsqu'il est vectorisé par le lipide cationique INTERFERin ;
- l'introduction de 1-3 spermines dans la molécule n'a que peu d'effet sur l'expression de la luciférase ;
- l'introduction de 20-30 spermines dans le siRNA conduit à une extinction comparable à celle obtenue en utilisant le vecteur INTERFERin.
- l'efficacité du siRNA avec 30 spermines augmente encore d'environ 10 fois en association avec un véhicule lipidique.

Il est tout à fait remarquable que ces effets sont observés à une concentration nanomolaire, c'est-à-dire 10.000 fois inférieure à celle utilisée dans les expériences effectuées avec les peptides TAT et pénétratine (9).

<u>Exemple 3 : ARN interférence ; extinction du gène de la luciférase par les siRNA cationiques. Effet de la concentration.</u>

La Figure 5 montre de haut en bas

- les contrôles du siRNA sans spermine vectorisé par INTERFERin montrant un effet à 1 nM ;
- l'expression de la luciférase GL3 n'est pas diminuée lorsque les cellules sont incubées avec le siRNA sans spermine

greffée et sans INTERFERin, quelle que soit la concentration ;

- une diminution concentration-dépendante de la luciférase en présence des siRNA comportant 20 et 30 spermines.

Exemple 4 : ARN interférence ; extinction du gène de la luciférase par les siRNA cationiques. Expériences de validation.

La Figure 6 valide les revendications et la spécificité de l'ARN interférence.

Après les contrôles négatif (100% d'expression des cellules seules) et positif (27% d'expression de la luciférase GL3 en présence du siRNA de séquence GL3 vectorisé par INTERFERin), le siRNA <GL3ss><GL3as>$S_1$ comportant cette fois une spermine greffée en position 5' du brin *antisens* a été testé.

Les barres 3 à 6 montrent que le greffage de la spermine en position 5' du brin antisens fait disparaître l'effet d'extinction de la luciférase. Il est en effet connu que cette position du siRNA doit être préalablement phosphorylée par la cellule pour pouvoir observer le phénomène d'ARN interférence.

Les barres 7 et 8 montrent que, même à une concentration 10 fois supérieure, le siRNA GL2 avec 20 spermines, comportant 3 mismatch par rapport à la cible GL3, ne diminue pas l'expression de la luciférase GL3.

Références Bibliographiques

1 BOLCATO-BELLEMIN, A.L., BONNET, M.E., CREUSAT, G., ERBACHER, P., and BEHR, J.P. (2007). Sticky overhangs enhance siRNA-mediated gene silencing. Proc Natl Acad Sci U S A 104, 16050-16055

2 KOPATZ, I., REMY, J.S., and BEHR, J.P. (2004). A model for non-viral gene delivery: through syndecan adhesion molecules and powered by actin. J Gene Med 6, 769-776

3 ZIMMERMANN, T.S., LEE, A.C., AKINC, A., BRAMLAGE, B., BUMCROT, D., FEDORUK, M.N., HARBORTH, J., HEYES, J.A., JEFFS, L.B., JOHN, M., JUDGE, A.D., LAM, K., MCCLINTOCK, K., NECHEV, L.V., PALMER, L.R., RACIE, T., ROHL, I., SEIFFERT, S., SHANMUGAM, S., SOOD, V., SOUTSCHEK, J., TOUDJARSKA, I., WHEAT, A.J., YAWORSKI, E., ZEDALIS, W., KOTELIANSKY, V., MANOHARAN, M., VORNLOCHER, H.P., and MACLACHLAN, I. (2006). RNAi-mediated gene silencing in non-human primates. Nature 441, 111-114

4 SANTEL, A., ALEKU, M., KEIL, O., ENDRUSCHAT, J., ESCHE, V., FISCH, G., DAMES, S., LOFFLER, K., FECHTNER, M., ARNOLD, W., GIESE, K., KLIPPEL, A., and KAUFMANN, J. (2006). A novel siRNA-lipoplex technology for RNA interference in the mouse vascular endothelium. Gene Ther 13, 1222-1234

5 KIM, S.H., JEONG, J.H., LEE, S.H., KIM, S.W., and PARK, T.G. (2006). PEG conjugated VEGF siRNA for anti-angiogenic gene therapy. Journal of Controlled Release 116, 123-129

6 MOSCHOS, S.A., WILLIAMS, A.E., and LINDSAY, M.A. (2007). Cell-penetrating-peptide-mediated siRNA lung delivery. Biochemical Society Transactions 35, 807-810

7 TURNER, J.J., JONES, S., FABANI, M.M., IVANOVA, G., ARZUMANOV, A.A., and GAIT, M.J. (2007). RNA targeting with peptide conjugates of oligonucleotides, siRNA and PNA. Blood Cells, Molecules, and Diseases 38, 1-7

8 demande PCT/IB2006/004085 ; WO2007/069092 ; inventeurs : J.P. Behr, M. Kotera, B. Pons, E. Voirin, et J.S. Remy

9 MOSCHOS, S.A., JONES, S.W., PERRY, M.M., WILLIAMS, A.E., ERJEFALT, J.S., TURNER, J.J., BARNES, P.J., SPROAT, B.S., GAIT, M.J., and LINDSAY, M.A. (2007). Lung delivery studies using siRNA conjugated to TAT(48-60) and penetratin reveal peptide induced réduction in gene expression and induction of innate immunity. Bioconjug Chem 18, 1450-1459


## Revendications

1. siRNA cationiques, **caractérisés en ce qu'**il s'agit de fragments d'ARN double brin, aux extrémités desquels sont greffés des oligocations, le nombre de charges cationiques greffées étant supérieur à celui des charges anioniques des phosphates internucléosidiques des brins d'ARN, et **caractérisés en ce que** lesdits oligocations sont des oligospermines greffées en 3' et/ou 5' du brin sens et/ou en 3' du brin antisens du siRNA conformément à la formule

$$(N)_i\text{-}\ [\overset{-}{PO_3}\text{-}(CH_2)_4\text{-}\overset{+}{NH_2}\text{-}(CH_2)_3\text{-}\overset{+}{NH_2}\text{-}(CH_2)_4\text{-}\overset{+}{NH_2}\text{-}(CH_2)_3\text{-}\overset{+}{NH_2}\text{-}(CH_2)_4\text{-}O]_jH$$

dans laquelle $(N)_i$ représente un oligoribonucléotide i-mère, avec i= 15 à 30 ainsi que toutes modifications chimiques ou substitutions conservant la forme A de la double hélice du siRNA et dans laquelle j = 1 à 50.

2. siRNA cationiques selon la revendication 1, **caractérisés en ce que** ledit i = 19 à 30.

3. siRNA cationiques selon la revendication 1 ou 2, **caractérisés en ce que** lesdites oligospermines sont greffées en 5' du brin sens.

4. siRNA cationiques selon l'une quelconque des revendications 1 à 3, **caractérisés en ce que** ledit $(N)_i$ représente un oligoribonucléotide i-mère avec i= 15 à 30.

5. siRNA cationiques selon l'une quelconque des revendications 1 à 4, **caractérisés en ce que** ledit j = 20 à 30.

6. siRNA cationiques selon l'une quelconque des revendications 1 à 5, **caractérisés en ce que** ledit j = 20 à 30 et **en ce que** ledit i = 21.

7. siRNA cationiques selon l'une quelconque des revendications 1 à 6, **caractérisés en ce que** la complémentarité des brins est totale ou partielle dans la double hélice.

8. siRNA cationiques selon l'une quelconque des revendications 1 à 7, **caractérisés en ce que** chaque oligoribonucléotide présente une extension simple brin en 3' de la double hélice.

9. siRNA cationiques selon l'une quelconque des revendications 1 à 8, **caractérisés en ce que** lesdits fragments sont constitués d'ARN double brin longs de 15 à 30 nucléotides aux extrémités desquels sont greffés, par liaison covalente 1 à 3 oligocations dont le nombre total de charges cationiques est supérieur au nombre de charges anioniques portées par l'ARN.

10. siRNA cationiques selon l'une quelconque des revendications 1 à 9, **caractérisés en ce que** lesdits fragments sont constitués d'ARN double brin longs de 19 à 30 nucléotides, aux extrémités desquels sont greffés, par liaison covalente

1 à 3 oligocations dont le nombre total de charges cationiques est supérieur au nombre de charges anioniques portées par l'ARN.

**11.** siRNA cationiques selon l'une quelconque des revendications 1 à 10, pour une utilisation comme médicaments.

**12.** Compositions pharmaceutiques, **caractérisées en ce qu'**elles renferment une quantité efficace d'au moins un siRNA cationique, selon l'une quelconque des revendications 1 à 11, en association avec un véhicule pharmaceutiquement inerte, un lipide cationique ou un polymère cationique.

**13.** Application des siRNA cationiques selon l'une quelconque des revendications 1 à 10, pour étudier la fonction ou la régulation de gènes cibles en biologie moléculaire et génomique fonctionnelle.

**14.** Procédé de synthèse automatisé d'un brin de siRNA cationique selon l'une quelconque des revendications 1 à 10, **caractérisé en ce qu'**il comprend le couplage séquentiel 3' vers 5' de 19 à 30 ribonucléotides sur support solide, précédé et/ou suivi du couplage séquentiel de synthons cationiques, le brin répondant à la formule (VI)

$$dT\text{-}dT\text{-}(N)_i\text{-}A_j \text{ ou } A_j\text{-}dT\text{-}dT\text{-}(N)_i \qquad (VI)$$

avec N, i et j tels que définis à la revendication 1, et avec A = $PO_3\text{-}(CH_2)_4\text{-}NH_2\text{-}(CH_2)_3\text{-}NH_2\text{-}(CH_2)_4\text{-}NH_2\text{-}(CH_2)_3\text{-}NH_2\text{-}(CH_2)_4\text{-}O$.

## Patentansprüche

**1.** Kationische siRNAs, **dadurch gekennzeichnet, dass** es sich um Fragmente von doppelsträngiger RNA handelt, an deren Enden Oligokationen aufgepfropft sind, wobei die Anzahl der aufgepfropften kationischen Ladungen höher als die der anionischen Ladungen der Internukleosidphosphate der RNA-Stränge ist, und **dadurch gekennzeichnet, dass** die Oligokationen Oligospermine sind, die am 3' und/oder am 5' des Sense-Stranges und/oder am 3' des Antisense-Stranges der siRNA entsprechend der Formel

$$(N)_i\text{-}[\overset{-}{PO_3}\text{-}(CH_2)_4\text{-}\overset{+}{N}H_2\text{-}(CH_2)_3\text{-}\overset{+}{N}H_2\text{-}(CH_2)_4\text{-}\overset{+}{N}H_2\text{-}(CH_2)_3\text{-}\overset{+}{N}H_2\text{-}(CH_2)_4\text{-}O]_jH$$

aufgepfropft sind, in der $(N)_i$ ein Oligoribonukleotid-i-mer mit i=15 bis 30, sowie alle chemischen Modifikationen oder Substitutionen, die die A-Form der Doppelhelix der siRNA konservieren, darstellt, und in der j = 1 bis 50.

**2.** Kationische siRNAs nach Anspruch 1, **dadurch gekennzeichnet, dass** i = 19 bis 30.

**3.** Kationische siRNAs nach Anspruch 1 oder 2, **dadurch gekennzeichnet, dass** die Oligospermine am 5' des Sense-Strangs aufgepfropft sind.

**4.** Kationische siRNAs nach einem beliebigen der Ansprüche 1 bis 3, **dadurch gekennzeichnet, dass** $(N)_i$ ein Oligoribonukleotid-i-mer mit i = 15 bis 30 darstellt.

**5.** Kationische siRNAs nach einem beliebigen der Ansprüche 1 bis 4, **dadurch gekennzeichnet, dass** j = 20 bis 30.

**6.** Kationische siRNAs nach einem beliebigen der Ansprüche 1 bis 5, **dadurch gekennzeichnet, dass** j = 20 bis 30, und dadurch, dass i = 21.

**7.** Kationische siRNAs nach einem beliebigen der Ansprüche 1 bis 6, **dadurch gekennzeichnet, dass** die Komplementarität der Stränge in der Doppelhelix vollständig oder partiell ist.

**8.** Kationische siRNAs nach einem beliebigen der Ansprüche 1 bis 7, **dadurch gekennzeichnet, dass** jedes Oligoribonukleotid eine einzelsträngige Erweiterung am 3' der Doppelhelix aufweist.

**9.** Kationische siRNAs nach einem beliebigen der Ansprüche 1 bis 8, **dadurch gekennzeichnet, dass** die Fragmente aus doppelsträngiger RNA gebildet sind, von 15 bis 30 Nukleotide lang, an deren Enden durch kovalente Bindung

1 bis 3 Oligokationen aufgepfropft sind, deren Gesamtzahl an kationischen Ladungen höher als die Zahl der anionischen Ladungen ist, die von der RNA getragen werden.

**10.** Kationische siRNAs nach einem beliebigen der Ansprüche 1 bis 9, **dadurch gekennzeichnet, dass** die Fragmente aus doppelsträngiger RNA gebildet sind, von 19 bis 30 Nukleotide lang, an deren Enden durch kovalente Bindung 1 bis 3 Oligokationen aufgepfropft sind, deren Gesamtzahl an kationischen Ladungen höher als die Zahl der anionischen Ladungen ist, die von der RNA getragen werden.

**11.** Kationische siRNAs nach einem beliebigen der Ansprüche 1 bis 10, zur Verwendung als Medikamente.

**12.** Pharmazeutische Zusammensetzungen, **dadurch gekennzeichnet, dass** sie eine wirksame Menge von mindestens einer kationischen siRNA gemäß einem beliebigen der Ansprüche 1 bis 11 in Assoziation mit einem inerten pharmazeutischen Träger, einem kationischen Lipid oder einem kationischen Polymer enthalten.

**13.** Anwendung der kationischen siRNAs gemäß einem beliebigen der Ansprüche 1 bis 10 zum Untersuchen der Funktion oder der Regulation von Zielgenen in der Molekularbiologie und der funktionellen Genomik.

**14.** Verfahren zur automatisierten Synthese eines Strangs von kationischer siRNA gemäß einem beliebigen der Ansprüche 1 bis 10, **dadurch gekennzeichnet, dass** es die sequentielle Kupplung von 3' nach 5' von 19 bis 30 Ribonukleotiden auf einem festen Träger umfasst, angeführt und/oder gefolgt von sequentieller Kupplung kationischer Synthons, wobei der Strang der Formel (VI) entspricht

$$\text{dT-dT-(N)}_i\text{-A}_j \text{ oder } \text{A}_j\text{-dT-dT-(N)}_i \qquad \text{(VI)}$$

mit N, i und j wie in Anspruch 1 definiert, und mit
$A = PO_3\text{-}(CH_2)_4\text{-}NH_2\text{-}(CH_2)_3\text{-}NH_2\text{-}(CH_2)_4\text{-}NH_2\text{-}(CH_2)_3\text{-}NH_2\text{-}(CH_2)_4\text{-}O$.

## Claims

**1.** A cationic siRNA, **characterized in that** it is a fragment of double-stranded RNA, at the ends of which oligocations are grafted, the number of grafted cationic charges being greater than that of the anionic charges on the internucleoside phosphates of the RNA strands, and **characterized in that** said oligocations are oligospermines grafted at the 3' and/or 5' end of the sense strand and/or at the 3' end of the antisense strand of the siRNA, in accordance with the formula:

$$(N)_i\text{-}[\overset{-}{PO_3}\text{-}(CH_2)_4\text{-}\overset{+}{NH_2}\text{-}(CH_2)_3\text{-}\overset{+}{NH_2}\text{-}(CH_2)_4\text{-}\overset{+}{NH_2}\text{-}(CH_2)_3\text{-}\overset{+}{NH_2}\text{-}(CH_2)_4\text{-}O]_jH$$

in which $(N)_i$ represents an i-meric oligoribonucleotide with i = 15 to 30, as well as any chemical modifications or substitutions that conserve the A form of the double helix of the siRNA and in which j = 1 to 50.

**2.** The cationic siRNA as claimed in claim 1, **characterized in that** said i = 19 to 30.

**3.** The cationic siRNA according to claim 1 or claim 2, **characterized in that** said oligospermines are grafted at the 5' end of the sense strand.

**4.** The cationic siRNA according to any one of claims 1 to 3, **characterized in that** said $(N)_i$ represents an i-meric oligoribonucleotide with i = 15 to 30.

**5.** The cationic siRNA according to any one of claims 1 to 4, **characterized in that** said j = 20 to 30.

**6.** The cationic siRNA according to any one of claims 1 to 5, **characterized in that** said j = 20 to 30 and **in that** said i = 21.

**7.** The cationic siRNA according to any one of claims 1 to 6, **characterized in that** the strand complementarity is total or partial in the double helix.

8. The cationic siRNA according to any one of claims 1 to 7, **characterized in that** each oligoribonucleotide has a single-strand extension at the 3' end of the double helix.

9. The cationic siRNA according to any one of claims 1 to 8, **characterized in that** said fragments are constituted by double-stranded RNA, which a length of 15 to 30 nucleotides, at the ends of which 1 to 3 oligocations are grafted by covalent bonding, the total number of cationic charges of said oligocations being greater than the number of anionic charges carried by the RNA.

10. The cationic siRNA according to any one of claims 1 to 9, **characterized in that** said fragments are constituted by double-stranded RNA, with a length of 19 to 30 nucleotides, at the ends of which 1 to 3 oligocations are grafted by covalent bonding, the total number of cationic charges of said oligocations being greater than the number of anionic charges carried by the RNA.

11. The cationic siRNA according to any one of claims 1 to 10, for use as a medicament.

12. A pharmaceutical composition, **characterized in that** it contains an effective quantity of at least one cationic siRNA according to any one of claims 1 to 11, in association with a pharmaceutically inert vehicle, a cationic lipid or a cationic polymer.

13. Application of cationic siRNAs according to any one of claims 1 to 10 to the study of the function or regulation of target genes in molecular biology and in functional genomics.

14. A process for the automated synthesis of a strand of cationic siRNA as claimed in any one of claims 1 to 10, **characterized in that** it comprises the sequential 3' to 5' coupling of 19 to 30 ribonucleotides to a solid support, preceded and/or followed by the sequential coupling of cationic synthons, the strand corresponding to the formula (VI)

$$dT\text{-}dT\text{-}(N)_i\text{-}A_j \text{ or } A_j\text{-}dT\text{-}dT\text{-}(N)_i \qquad (VI)$$

in which N, i and j are as defined in claim 1, and in which A = $PO_3\text{-}(CH_2)_4\text{-}NH_2\text{-}(CH_2)_3\text{-}NH_2\text{-}(CH_2)_4\text{-}NH_2\text{-}(CH_2)_3\text{-}NH_2\text{-}(CH_2)_4\text{-}O$.

Figure 1

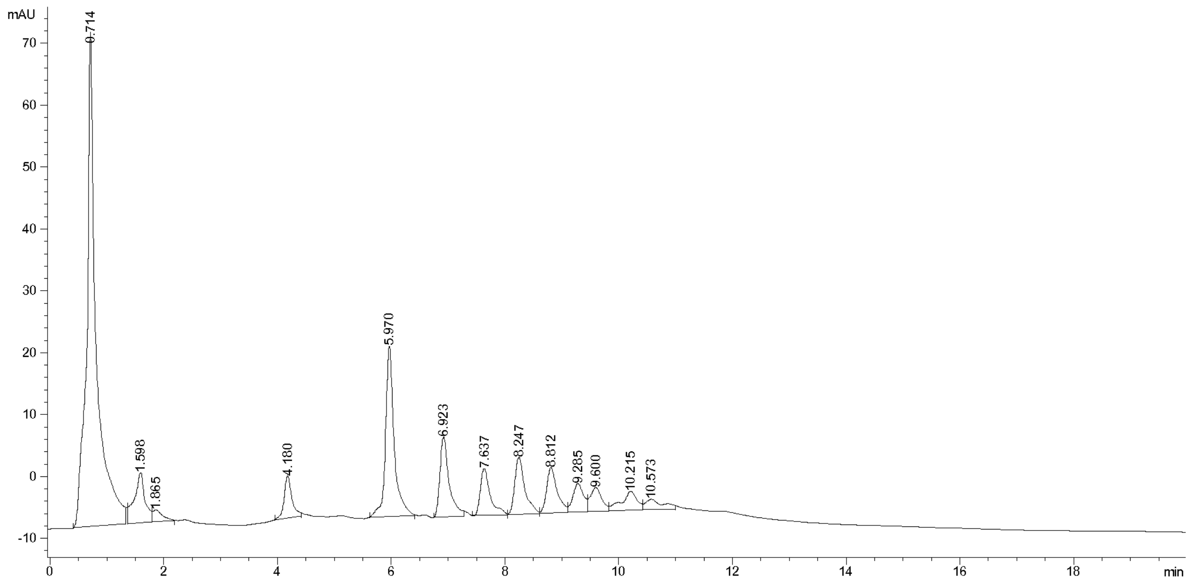


<GL3>$S_5$ décomposé

Figure 2

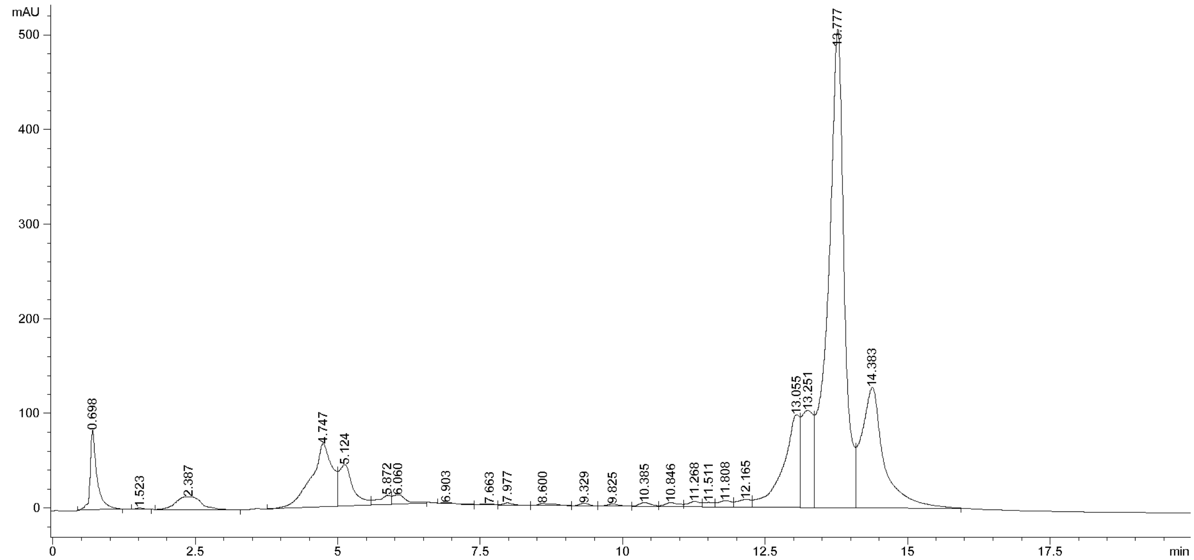


<GL3ss>

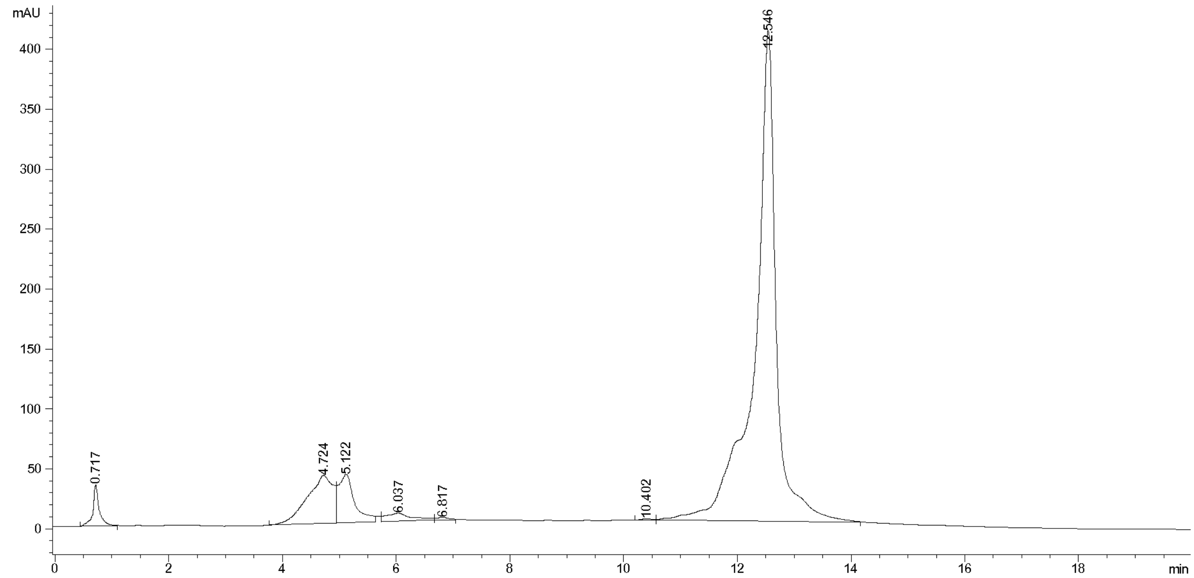


<GL3ss>$S_1$

Figure 2 (suite)

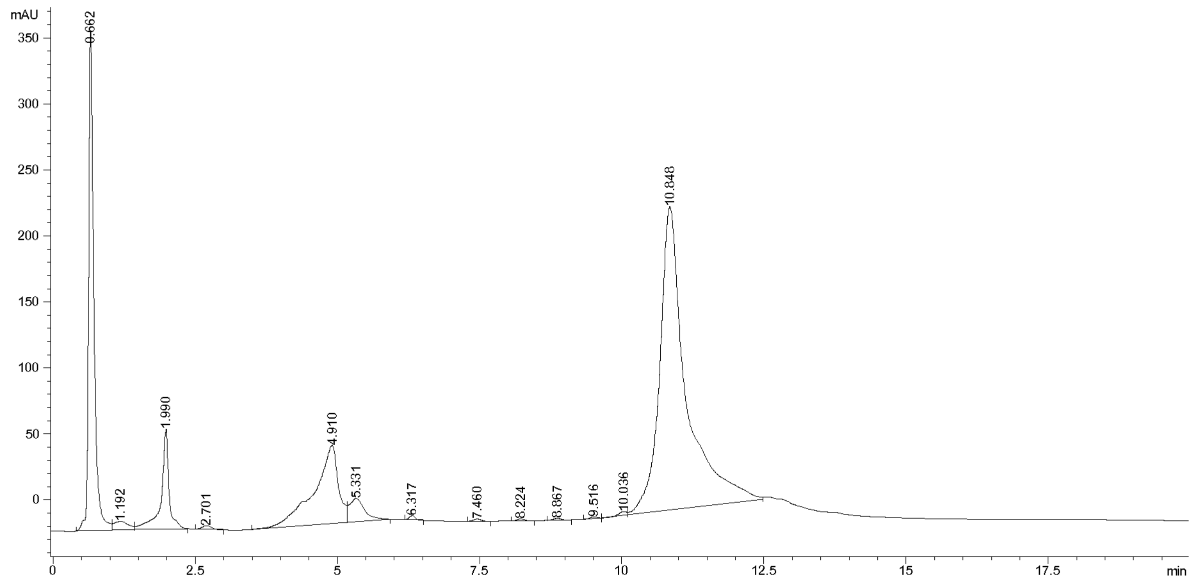


<GL3ss>$S_3$

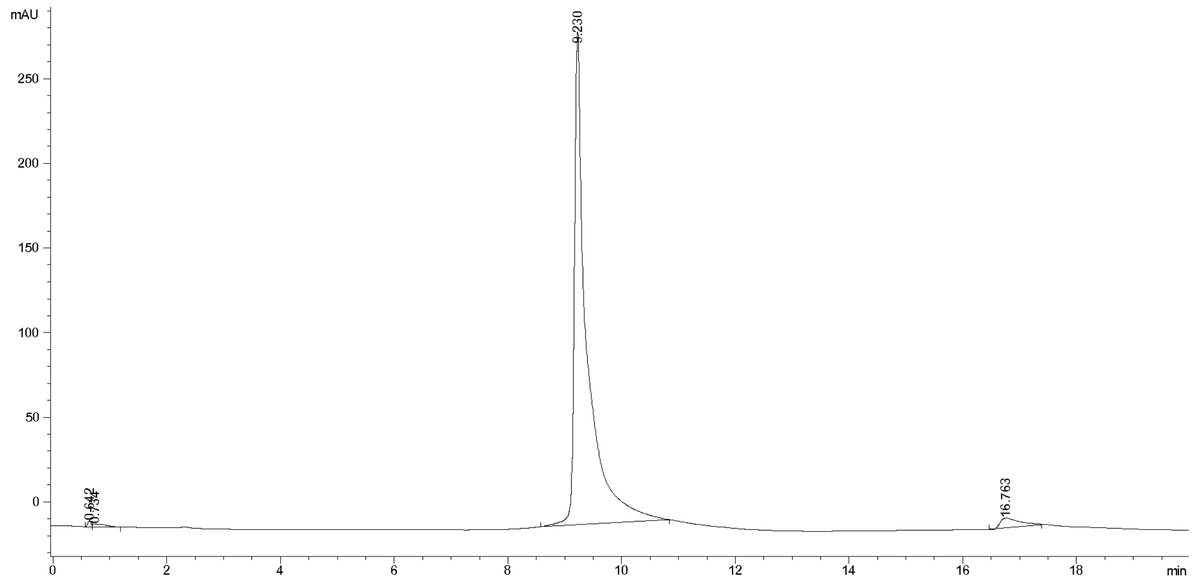


<GL3ss>$S_5$

Figure 2 (suite)

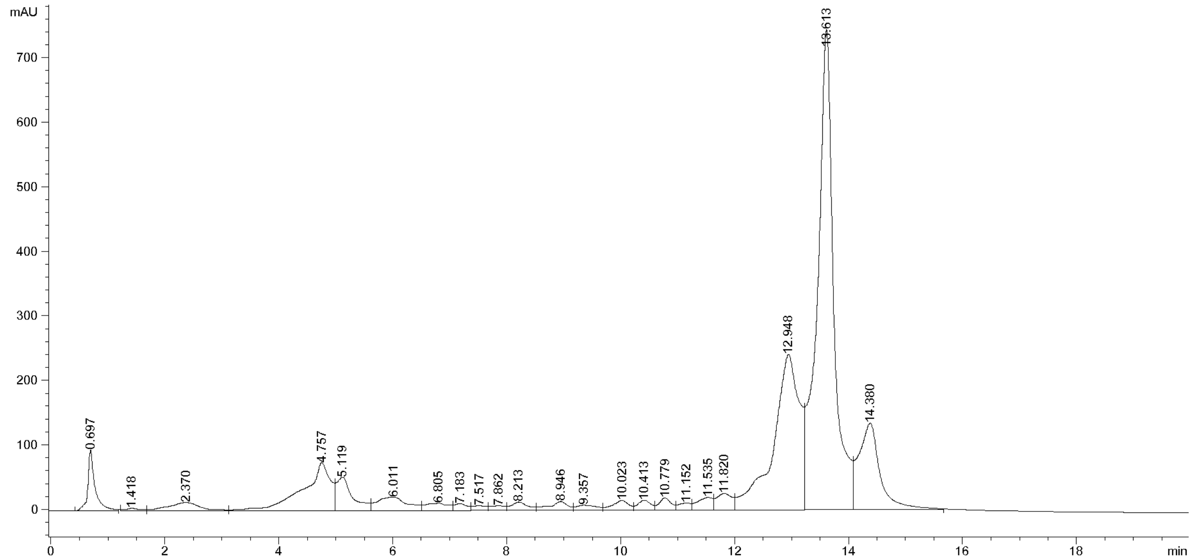


<GL3as>

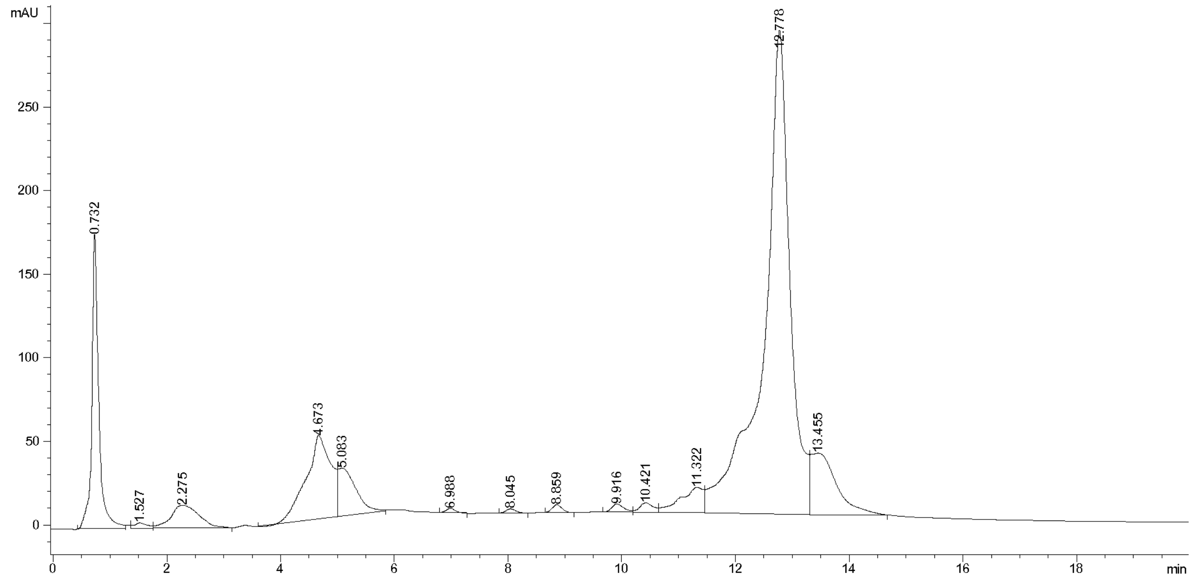


<GL3as>$S_1$

Figure 3

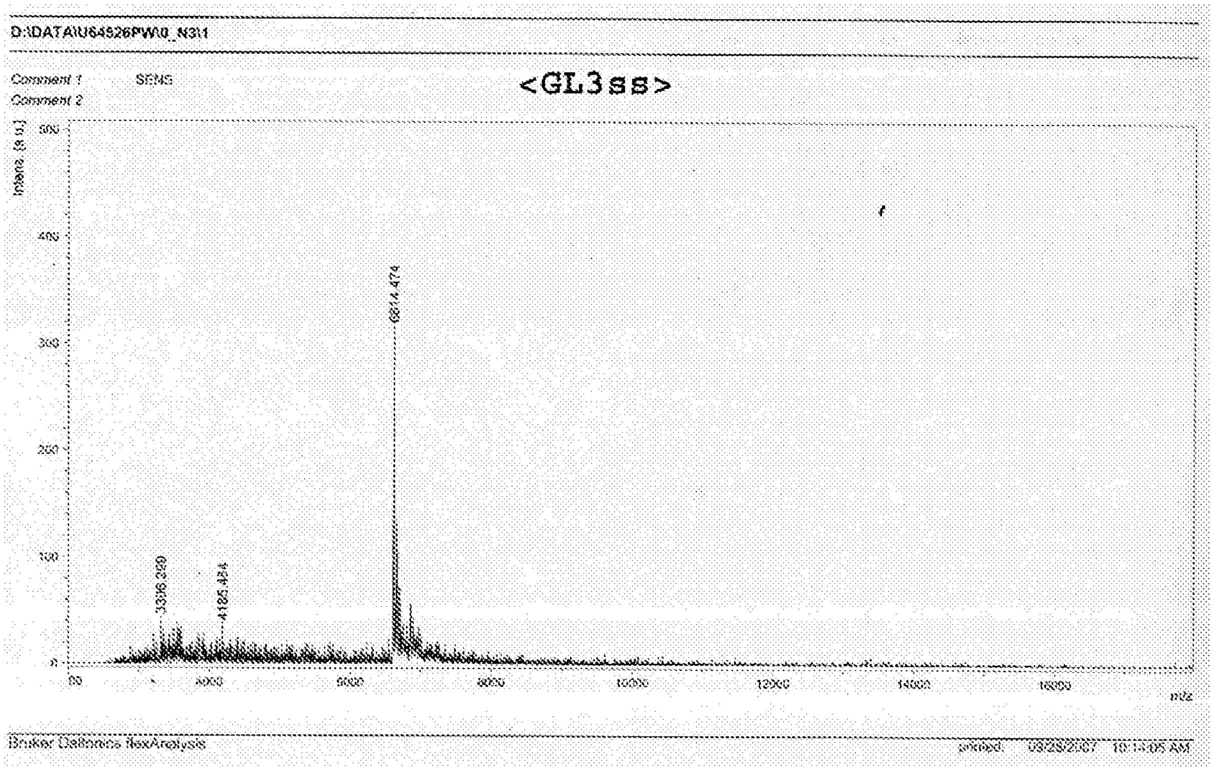

D:\DATA\U64526PW\0_N3\1
Comment 1 SENS
Comment 2
<GL3ss>
Intens. [a.u.]
6214.474
3396.329
4185.484
m/z
Bruker Daltonics flexAnalysis


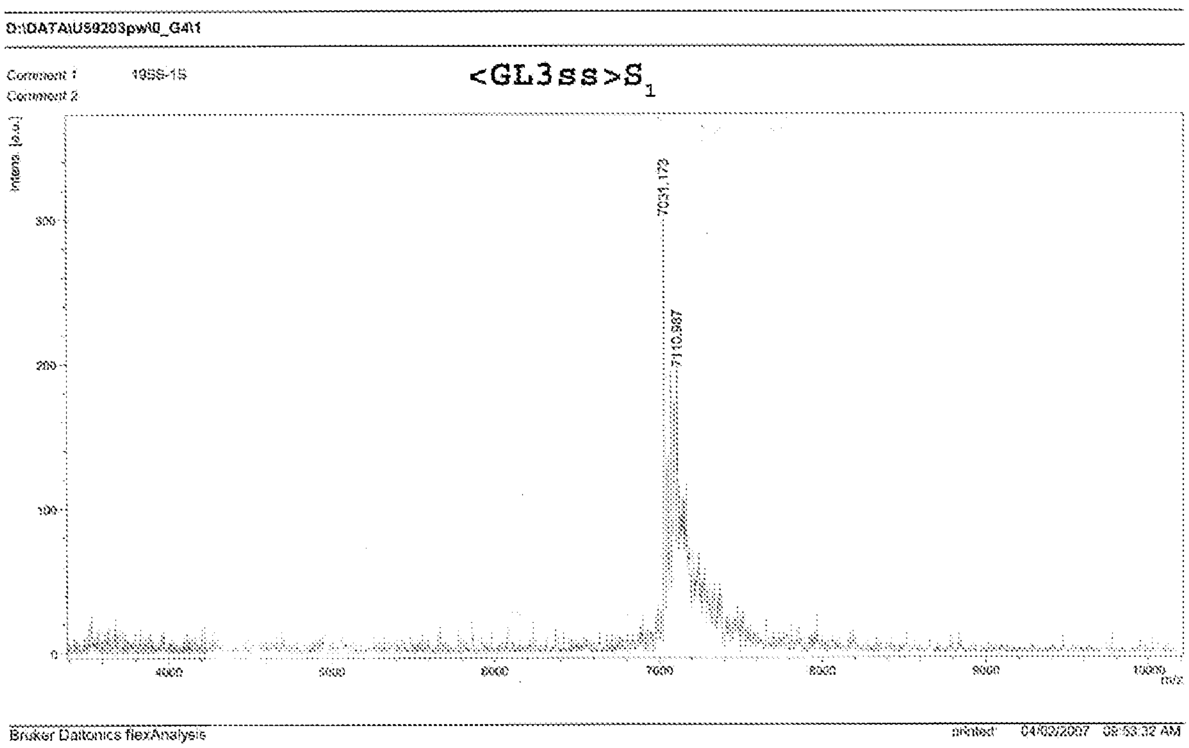

D:\DATA\U59203pw\0_G4\1
Comment 1 19SS-15
Comment 2
<GL3ss>S1
Intens. [a.u.]
7031.172
7110.887
m/z
Bruker Daltonics flexAnalysis

Figure 3 (suite)

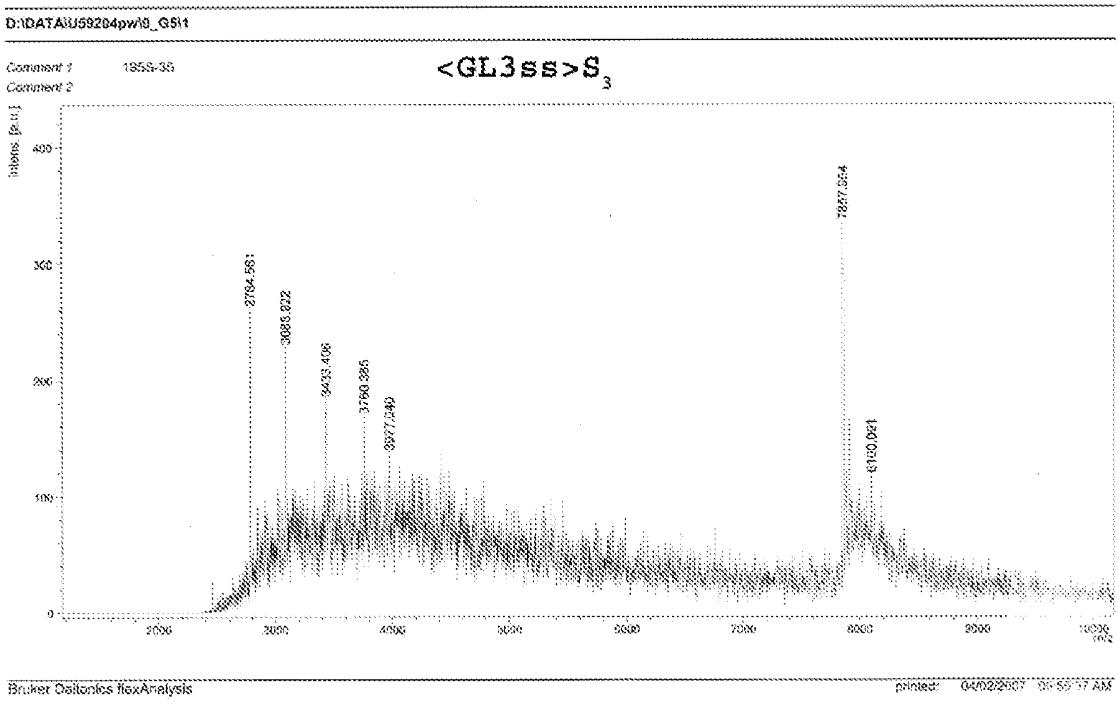


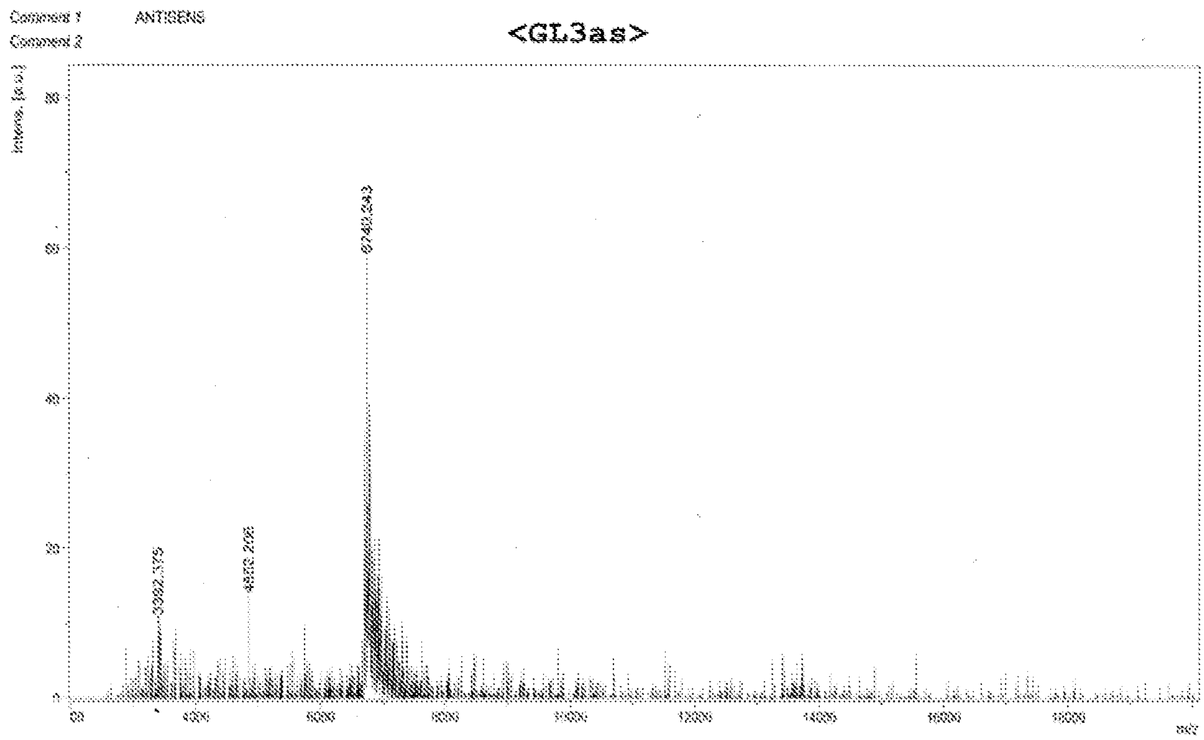

Figure 3 (suite)

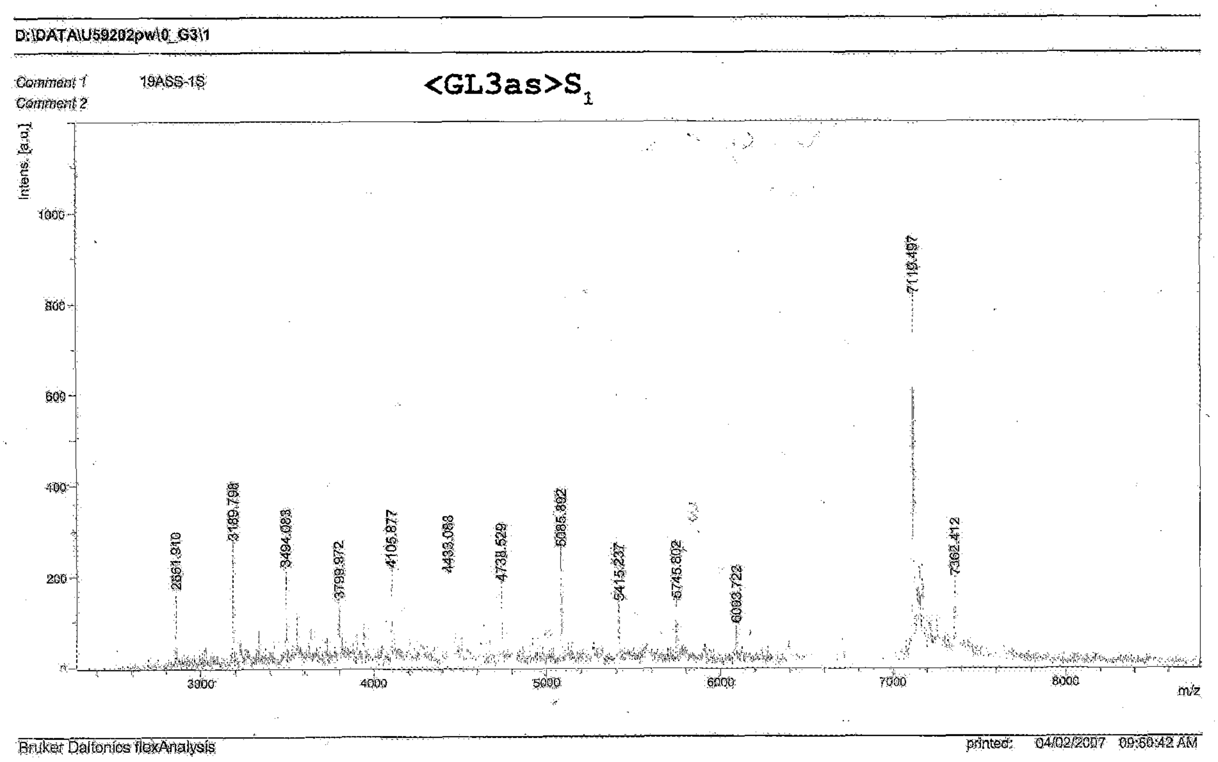


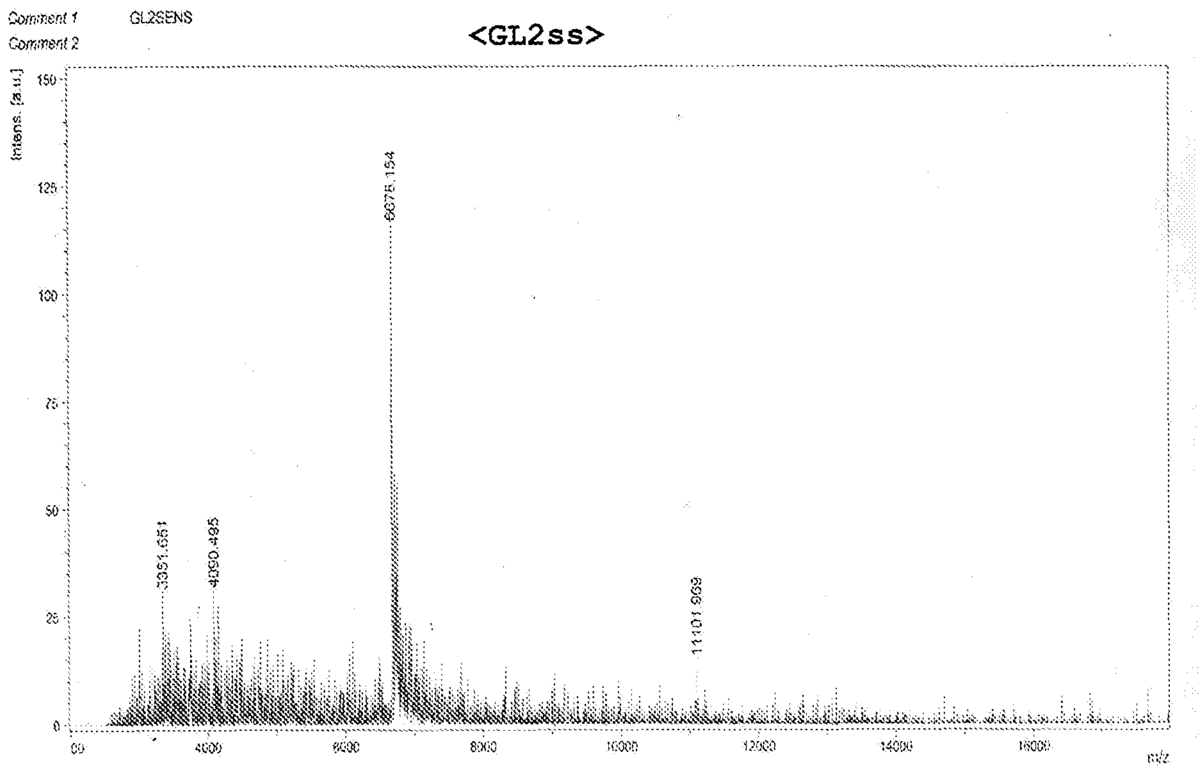

Figure 4

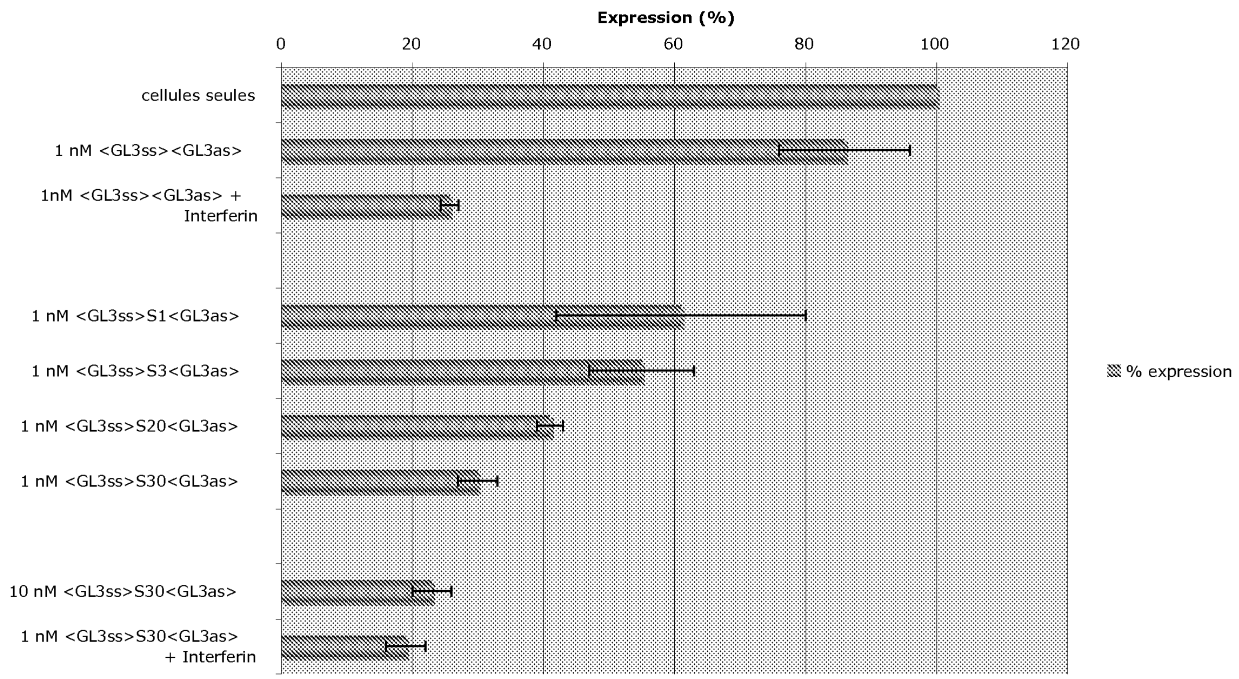


Figure 5

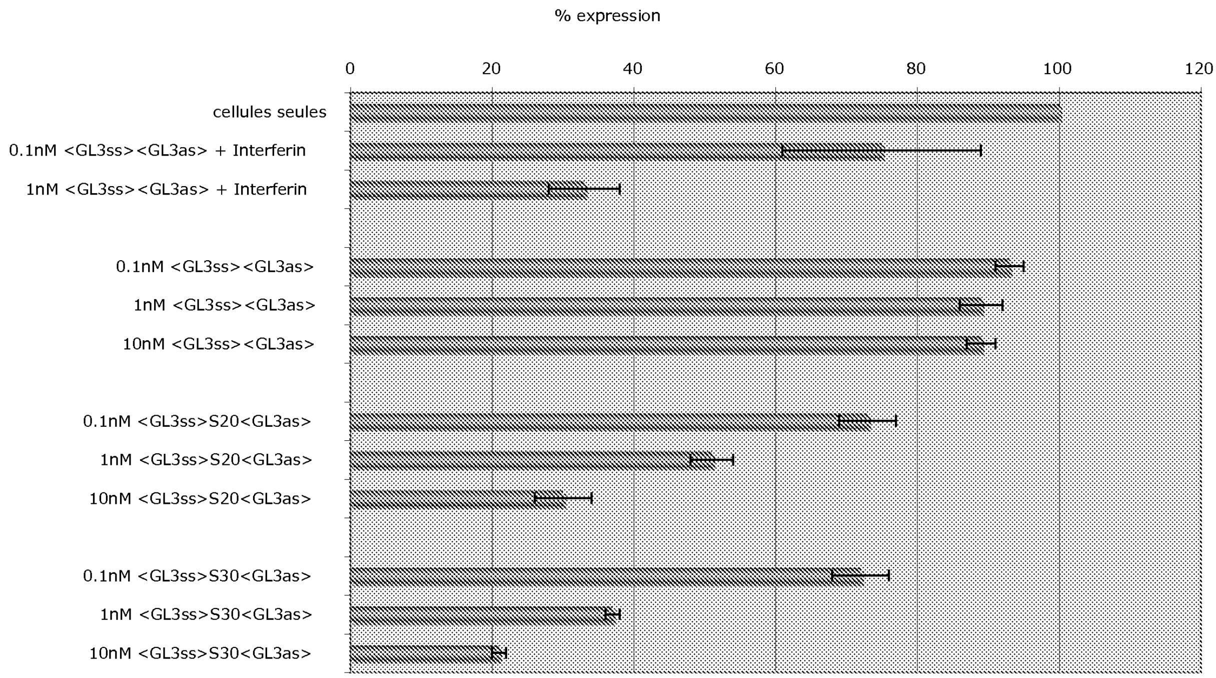

Figure 6

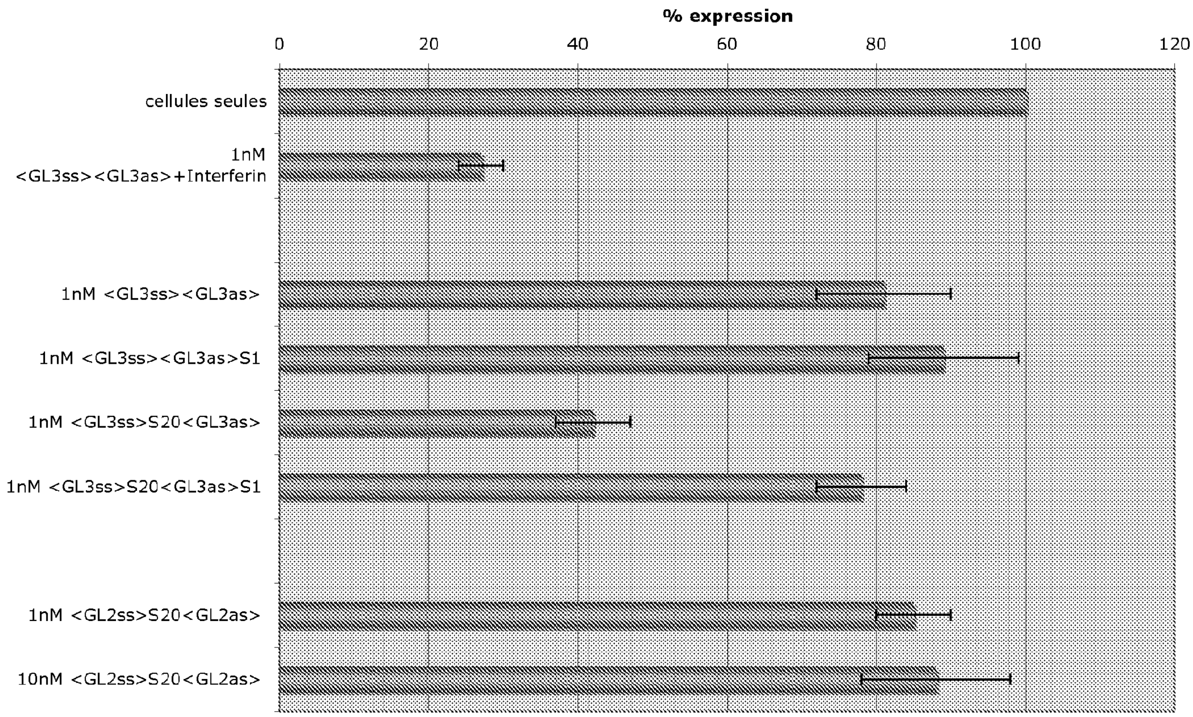
% expression
0
20
40
60
80
100
120
cellules seules
1nM <GL3ss><GL3as>+Interferin
1nM <GL3ss><GL3as>
1nM <GL3ss><GL3as>S1
1nM <GL3ss>S20<GL3as>
1nM <GL3ss>S20<GL3as>S1
1nM <GL2ss>S20<GL2as>
10nM <GL2ss>S20<GL2as>

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006052854 A
- WO IB2006004085 A
- WO 2007069092 A
- FR 627663238
- FR IB2009050379 W
- FR 0800492


### Littérature non-brevet citée dans la description


- **BOLCATO-BELLEMIN, A.L. ; BONNET, M.E. ; CREUSAT, G. ; ERBACHER, P. ; BEHR, J.P.** Sticky overhangs enhance siRNA-mediated gene silencing. *Proc Natl Acad Sci U S A,* 2007, vol. 104, 16050-16055
- **KOPATZ, I. ; REMY, J.S. ; BEHR, J.P.** A model for non-viral gene delivery: through syndecan adhesion molecules and powered by actin. *J Gene Med,* 2004, vol. 6, 769-776
- **ZIMMERMANN, T.S. ; LEE, A.C. ; AKINC, A. ; BRAMLAGE, B. ; BUMCROT, D. ; FEDORUK, M.N. ; HARBORTH, J. ; HEYES, J.A. ; JEFFS, L.B. ; JOHN, M.** RNAi-mediated gene silencing in non-human primates. *Nature,* 2006, vol. 441, 111-114
- **SANTEL, A. ; ALEKU, M. ; KEIL, O. ; ENDRUSCHAT, J. ; ESCHE, V. ; FISCH, G. ; DAMES, S. ; LOFFLER, K. ; FECHTNER, M. ; ARNOLD, W.** A novel siRNA-lipoplex technology for RNA interference in the mouse vascular endothelium. *Gene Ther,* 2006, vol. 13, 1222-1234
- **KIM, S.H. ; JEONG, J.H. ; LEE, S.H. ; KIM, S.W. ; PARK, T.G.** PEG conjugated VEGF siRNA for anti-angiogenic gene therapy. *Journal of Controlled Release,* 2006, vol. 116, 123-129
- **MOSCHOS, S.A. ; WILLIAMS, A.E. ; LINDSAY, M.A.** Cell-penetrating-peptide-mediated siRNA lung delivery. *Biochemical Society Transactions,* 2007, vol. 35, 807-810
- **TURNER, J.J. ; JONES, S. ; FABANI, M.M. ; IVANOVA, G. ; ARZUMANOV, A.A. ; GAIT, M.J.** RNA targeting with peptide conjugates of oligonucleotides, siRNA and PNA. *Blood Cells, Molecules, and Diseases,* 2007, vol. 38, 1-7
- **MOSCHOS, S.A. ; JONES, S.W. ; PERRY, M.M. ; WILLIAMS, A.E. ; ERJEFALT, J.S. ; TURNER, J.J. ; BARNES, P.J. ; SPROAT, B.S. ; GAIT, M.J. ; LINDSAY, M.A.** Lung delivery studies using siRNA conjugated to TAT(48-60) and penetratin reveal peptide induced réduction in gene expression and induction of innate immunity. *Bioconjug Chem,* 2007, vol. 18, 1450-1459

SEQUENCE LISTING

<110> Centre National de la Recherche Scientifique

<120> siRNA CATIONIQUES, SYNTHESE ET UTILISATION POUR L'ARN INTERFERENCE

<130> CP/BB 62766-3238

<140> PCT/IB2009/050379
<141> 2009-01-30

<150> FR 08/00492
<151> 2008-01-30

<160> 5

<170> PatentIn version 3.5

<210> 1
<211> 14
<212> PRT
<213> Artificial Sequences

<220>
<223> Peptide

<400> 1

```
Gly Arg Lys Lys Arg Arg Gln Arg Arg Arg Pro Pro Gln Cys
1               5                   10
```

<210> 2
<211> 17
<212> PRT
<213> Artificial Sequences

<220>
<223> Peptide

<400> 2

```
Arg Gln Ile Lys Ile Trp Phe Gln Asn Arg Arg Met Lys Trp Lys Lys
1               5                   10                  15

Cys
```

<210> 3
<211> 19
<212> RNA
<213> Artificial Sequences

<220>
<223> oligonucleotide

<400> 3
agcuucauga gucgcauuc 19

<210> 4
<211> 19
<212> RNA
<213> Artificial Sequences

<220>
<223> Plasmide

<400> 4
agcuucauaa ggcgcaugc 19

<210> 5
<211> 19
<212> RNA
<213> Artificial Sequences

<220>
<223> Antisens

<400> 5
gaaugcgacu caugaagcu 19